# EUROPÄISCHE PATENTSCHRIFT

(11) **EP 1 455 650 B1**
(45) Veröffentlichungstag und Bekanntmachung des Hinweises auf die Patenterteilung: **12.12.2007**
(21) Anmeldenummer: 02796509.4
(22) Anmeldetag: 16.12.2002
(51) Int. Cl.: A61B 5/15

(54) **BLUTENTNAHMEVORRICHTUNG**
BLOOD-COLLECTION DEVICE
DISPOSITIF DE PRELEVEMENT DU SANG

(30) Priorität: 21.12.2001 DE 10163719
(43) Veröffentlichungstag der Anmeldung: 15.09.2004
(73) Patentinhaber: Sarstedt AG & Co., 51588 Nümbrecht (DE)
(72) Erfinder: SARSTEDT, Walter, 51588 Nümbrecht (DE)
(74) Vertreter: Grosse, Wolf-Dietrich Rüdiger
(86) Internationale Anmeldenummer: PCT/DE2002/004587
(87) Internationale Veröffentlichungsnummer: WO 2003/055392

(56) Entgegenhaltungen:
- CA-A- 1 333 463
- US-A- 4 240 425
- US-A- 4 378 812
- US-A- 5 755 701
- PATENT ABSTRACTS OF JAPAN vol. 1996, no. 05, 31. Mai 1996 (1996-05-31) & JP 08 000600 A (NISSHO CORP), 9. Januar 1996 (1996-01-09)

## Beschreibung

Die Erfindung betrifft eine Vorrichtung zur Aufnahme von Körperflüssigkeiten, mit einem Röhrchen, das an seinem vorderen Ende einen Dom mit einem durchstechbaren Stopfen für eine auf den Dom aufsteckbare Führungshülse trägt, die auf der dem Dom zugewandten Seite eine Kanüle mit einem Ventilgummi und auf der dem Dom abgewandten Seite ein Verbindungsstück oder den vorderen Teil einer Doppelkanüle aufweist.

Solche Vorrichtungen werden z.B. benötigt sowohl zur Blutentnahme aus einem Blutgefäß oder aus einem Blutspendebeutel als auch zur Probennahme aus einem Sammelbehälter für beispielsweise Urin. Hierbei tritt in allen Fällen die Schwierigkeit auf, daß das die Kanüle umschließende bzw. einhüllende, sich beim Zusammenfügen bzw. Aufstecken der Führungshülse auf den Dom ziehharmonikaartig zusammenschiebende Ventilgummi eine der Haltekraft zwischen Führungshülse und Dom entgegenwirkende Feder- bzw. Rückstellkraft ausübt, die dazu führen kann, daß sich die Führungshülse von dem Dom der Kappe abdrückt. Um dies zu vermeiden, müssen Gegenmaßnahmen getroffen werden.

Bei einer aus der DE 30 49 503 C bekannten Blutentnahmevorrichtung besitzt die das Entnahmeröhrchen an seinem vorderen Ende abschließende Kappe einen zylindrischen, in axialer Richtung vorstehenden Dom. Der Dom ist an seinem vorderen Ende durch einen durchstechbaren Verschlußstopfen abgeschlossen, der auf einer mit einer Mittelbohrung versehenen Stirnplatte des Doms aufliegt und von einem am vorderen Ende umgebördelten Kragen gehalten wird. Die rohrförmige Führungshülse, die an ihrem vorderen Ende in einem Halter eine doppelendige, beiderseits mit einer Schneidkante versehene Kanüle trägt, deren aus der Führungshülse vorstehendes Ende zur Einführung in eine Vene dient, während ihr hinteres Ende so weit in die Führungshülse hineinragt, daß es beim Ansetzen der Führungshülse an das Entnahmeröhrchen den Verschlußstopfen durchsticht, ist axial verschiebbar und drehbar auf dem Dom angeordnet. Das hintere, in die Führungshülse hineinragende Kanülenende wird von einem sackartigen Schlauch (Ventilgummi) solcher Länge eingehüllt, daß die Schneidkante des hinteren Kanülenendes bei gestrecktem Schlauch noch nicht dessen Boden berührt.

Damit die Führungshülse trotz der Federkraft des Ventilgummis in ihrer Position auf dem Dom gehalten wird, ist zur Verbindung der Doppelkanüle mit dem Dom letzterer mit einem seitlich vorstehenden Haltenocken versehen, dem winkelartige Schlitzausnehmungen in der Führungshülse zugeordnet sind. Mittels des in einen der über den Umfang verteilten Schlitze eingeführten Haltenockens läßt sich eine bajonettverschlußartige Drehverriegelung der in lockerer Passung auf dem Dom sitzenden Führungshülse der Doppelkanüle erreichen. Eine derartige Drehverriegelung gewährleistet einen sehr sicheren Zusammenhalt der zusammengefügten Teile der Blutentnahmevorrichtung, erhöht allerdings deren Herstellungsaufwand. Außerdem setzt das Ankuppeln bzw. Aufsetzen und Verriegeln der Führungshülse voraus, daß der Haltenocken zunächst durch Drehen des Entnahmeröhrchens mit der verschließenden Schraub- oder Stopfenkappe in Flucht mit einem Einführschlitz gebracht werden muß, was eine suchende Handhabung erfordert, weil die Teile gezielt gegeneinander ausgerichtet werden müssen.

Aus der DE 692 25 609 T2 ist ein Schutzgehäuse für eine in einen Kanülenhalter eingeschraubte Kanüle bekannt. Damit sich das Schutzgehäuse um den Halter verdrehen läßt, ist das Schutzgehäuse mit einem innen eine umlaufende Nut aufweisenden Ring formschlüssig auf einer an einem Dom des Halters ausgebildeten Wulst angeordnet.

Der Erfindung liegt die Aufgabe zugrunde, eine Vorrichtung der eingangs genannten Art mit einer einfachen und bedienungssicheren Steckverbindung für die beiden zusammenzufügenden Teile zu schaffen, die einen geringeren Herstellungsaufwand erfordert und bei gleichzeitig einfacherer Handhabung eine Haltekraft zwischen Führungshülse und Dom ermöglicht, die größer ist als die entgegenwirkende Federkraft (Rückstellkraft) des Ventilgummis.

Diese Aufgabe wird erfindungsgemäß für eine erste Ausführung mit den im Anspruch 1, für eine zweite Ausführung mit den im Anspruch 2 und für eine weitere Ausführung mit den im Anspruch 3 angegebenen Merkmalen gelöst.

In allen Fällen wird von der Erkenntnis ausgegangen, daß z.B. als Haltemittel übliche Längsrippen zwar eine Haftung zwischen der Führungshülse und dem Dom der Stopfen- oder Schraubkappe ermöglichen, jedoch keinen sicheren Sitz - entweder zu stramm oder aufgrund nicht genügender Vorspannung zu lose - der beiden zusammengesteckten Teile der Vorrichtung gewährleisten, so daß eine ausreichende, beim Abkoppeln eines gefüllten und Ankoppeln eines folgenden Entnahmeröhrchens nicht nachteilige Haftung eher zufällig ist. Diese wird hingegen aufgrund der erfindungsgemäßen Maßnahmen zuverlässig erreicht, wenn z.B. der in der Ausgangslage vor dem Aufschieben der Führungshülse in dem späteren Verbindungsbereich mit einem Übermaß, aufgrund z.B. einer bauchigen oder tonnenförmigen Außenkontur des Doms oder Innenkontur der Führungshülse (vgl. Anspruch 2) bzw. diese beiden Maßnahmen in Kombination miteinander, alternativ bedingt durch das vorstehende, mindestens eine Haltemittel (wie eine Längsrippe, Wulst, Nocken oder dergleichen), ausgebildete Dom so beschaffen ist, beispielsweise durch eine dünnere Wanddicke oder ein weniger steifes Material, daß er von der beim Aufschieben der harten, verformungssteifen Führungshülse bewirkten und über das radial abstehende Haltemittel bzw. die vorstehende Kontur übertragenen Kraft elastisch nach innen ausweicht, dabei aber gleichwohl auf die Führungshülse eine Kraft ausübt, die die Führungshülse gegen die Rückstell- bzw. Federkraft auf dem Dom festhält.

Bei der weiteren erfindungsgemäßen Ausführung verlagert sich das von dem Dommantel ab- bzw. vorstehende Haltemittel elastisch ausweichend zur Seite, indem sich die Längsrippen oder stattdessen z.B. eng aneinandergereihte Nocken bzw. kurze Stege oder dergleichen Haltemittel um ihre Längsachse seitlich in Umfangsrichtung des Doms wegdrücken. Damit läßt sich trotz der relativ kleinen Durchmesser, die die Steckverbinderteile (Führungshülse und Dom) aufweisen, der gewünschte selbstklemmende Halt erreichen, wobei sich die Teile gleichzeitig aber leichter und ruckfreier zusammenstecken und lösen lassen als eine herkömmliche Luer-Konus-Verbindung.

Die durch das Ausweichen des Doms bzw. seine sich im Bereich der Haltemittel oder der vorstehenden Kontur von Dom und/oder Führungshülse aufgrund der Krafteinwirkung durch die steife Führungshülse nach innen verformende Domwandung oder seines zumindest bereichsweise aus seiner normalen Orientierungslage zur Seite hin ausweichenden bzw. sich wegdrückenden Haltemittels bewirkte Klemmung mit Vorspannung, d.h. die Haltekraft ist dabei so, daß die Rückstellkraft des Ventilgummis die Führungshülse nicht von dem Dom abdrückt. Die aufgrund der Durchmesser-Verhältnisse erzielte temporäre radiale Ausweich-Verformung entweder des Doms oder nur das seitliche Ausweichen des Haltemittels jeweils unter der Kraft der verformungssteifen Führungshülse läßt sich anhand der Materialeigenschaften durch entsprechende Abstimmung der Maße und Elastizität festlegen.

Es wird somit stets das Zusammenspiel einer verformungssteifen Führungshülse mit dem Dom und ein zwischen diesen Teilen vorgesehenes Durchmesserübermaß verwirklicht, damit sich im Verbindungsbereich aufgrund eines kraftschlüssigen Verbundes die Haltekraft erzielen läßt. Dies läßt sich erreichen, indem der Dom von einer solchen nachgiebigen, elastisch verformbaren Beschaffenheit, beispielsweise hinsichtlich des verwendeten Kunststoffs und/oder der Bemessung seiner Wanddicke ist, daß seine Wandung radial nach innen ausweicht, wenn die Führungshülse aufgeschoben wird. Das Übermaß und Ausweichen des elastisch nachgiebig ausgebildeten Doms läßt sich gleichermaßen dadurch erreichen, daß der Innendurchmesser der Führungshülse, die z.B. Längsrippen, eine Wulst oder eine sonstige partielle Erhöhung aufweisen kann, kleiner als der Außendurchmesser des Doms ist, der dann zylindrisch sein kann und gleichwohl unter der Kraft der Führungshülse radial nach innen ausweicht. Hingegen ist bei seitlich ausweichendem Haltemittel der Dom ebenso verformungssteif wie die Führungshülse.

Weitere Merkmale und Einzelheiten der Erfindung ergeben sich aus den Patentansprüchen und der nachfolgenden Beschreibung von in den Zeichnungen anhand einer Haltemittel am Dom der Röhrchen-Verschlußkappe aufweisenden Vorrichtung zur Entnahme von Blut dargestellten Ausführungsbeispielen des Gegenstandes der Erfindung. Es zeigen:
- Fig. 1: ein Entnahmeröhrchen mit Führungshülse, explosiv dargestellt;
- Fig. 2: eine Gesamtansicht der auf einen Dom des Entnahmeröhrchens nach Fig. 1 aufgesteckten Führungshülse;
- Fig. 3: eine andere Ausführung von Entnahmeröhrchen und Führungshülse, explosiv dargestellt;
- Fig. 4: einen Schnitt entlang der Linie IV-IV von Fig. 3;
- Fig. 5: als Gesamtansicht die auf einen Dom des Entnahmeröhrchens nach Fig. 3 aufgesteckte Führungshülse; und
- Fig. 6: einen Schnitt entlang der Linie VI-VI von Fig. 5.

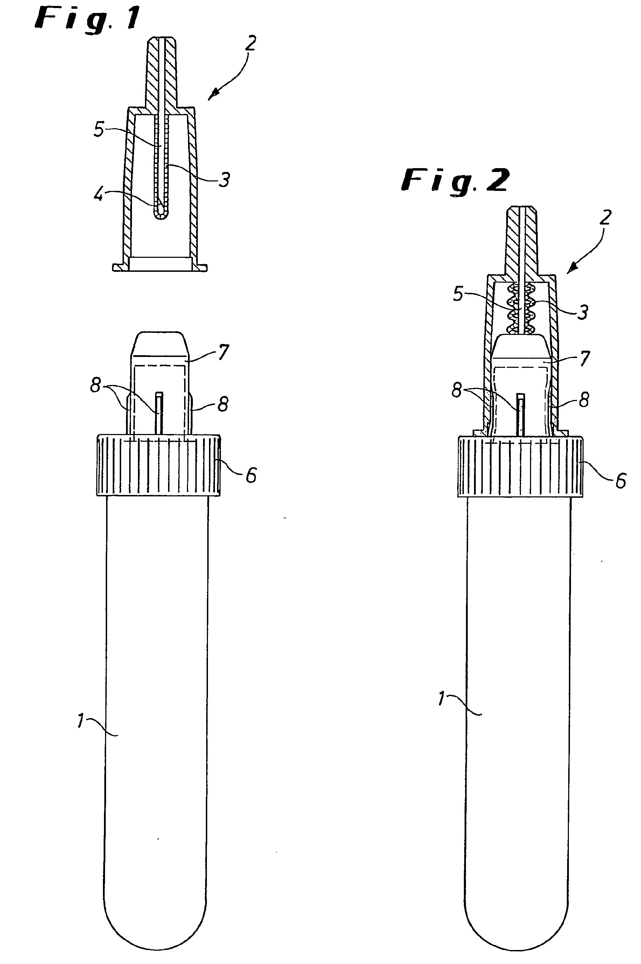

Eine in den Fig. 1 und 2 dargestellte Blutentnahmevorrichtung umfaßt ein Entnahmeröhrchen 1 mit einem Dom 7 bzw. 107 und eine - in allen Ausführungen beispielsweise mit einem Luer-Konus gezeigte - Führungshülse 2, die eine von einem Ventilgummi 3 umschlossene, eine angeschärfte Schneidkante 4 aufweisende Kanüle 5 aufnimmt. Bei anderen Varianten einer Führungshülse ist auf der von dem Dom abgewandten Seite ein Verbindungsstück oder der vordere Teil einer Doppelkanüle vorgesehen. Die Führungshülse 2 besteht aus einem verformungssteifen, harten Kunststoffmaterial. Das Entnahmeröhrchen 1 ist oben mit einer Kappe 6 verschlossen, die mit einem zylindrischen Dom 7 versehen ist. Dieser besitzt im Ausführungsbeispiel an seinem Außenumfang bzw. seiner Domwandung über den Umfang verteilt mehrere Haltemittel 8, die hier in Form von Längsrippen ausgebildet sind, die sich beginnend etwa ab der Mitte der Domlänge bis zum Domfuß hin erstrecken. Alternativ könnten als Haltemittel beispielsweise auch Reihen von untereinander eng an eng liegenden Nocken oder dergleichen vorgesehen bzw. gleichwirkende Ausgestaltungen der Domaußenkontur und/oder der Innenkontur der Führungshülse, z.B. bauchig oder tonnenartig, vorgenommen werden. In dem von der vorstehenden Kontur bzw. von den Haltemitteln 8 definierten Verbindungsbereich liegt ein Durchmesserübermaß vor, d.h. der Außendurchmesser des Doms 7 ist größer als der Innendurchmesser der Führungshülse 2. Ein solches Durchmesserübermaß in Relation von Führungshülse 2 und Dom 7 liegt gleichermaßen dann vor, wenn abweichend von der zeichnerischen Darstellung der Innendurchmesser der Führungshülse partiell kleiner als der Außendurchmesser des Doms ist.

Zur Blutentnahme werden die Führungshülse 2 und das Entnahmeröhrchen 1 durch Stecken miteinander verbunden, d.h. die verformungssteife Führungshülse 2 wird auf den Dom 7 der Stopfenkappe 6 aufgeschoben. Dabei trifft zunächst der Boden des Ventilgummis 3 auf den vertieft, zurückversetzt im Dom 6 angeordneten Stopfen auf. Beim weiteren Aufstecken schiebt sich das Ventilgummi 3 zieharmonikaartig zusammen (vgl. Fig. 2), wird dann von der Schneidkante 4 des hinteren Kanülenendes 5 durchstochen, die danach den Stopfen durchdringt, so daß die Schneidkante 4 frei im Inneren des Entnahmeröhrchens 1 liegt. Sobald die verformungssteife Führungshülse 2 dann in bis zu ihrer Endposition nach Fig. 2 zunehmendem Kontakt mit den Haltemitteln 8 (hier Längsrippen) gelangt, wird unter der sich über die Haltemittel 8 übertragenden Kraft der stabilen Führungshülse 2 die demgegenüber elastisch nachgebende Wandung des Doms 7 in diesem Abschnitt der Krafteinwirkung aufgrund der aufgeschobenen Führungshülse 2 radial nach innen ausweichend in eine eingeschnürte Form, wie aus Fig. 2 zu erkennen, gebracht. Die Führungshülse 2 sitzt danach unter Vorspannung mit der gewünschten Haltekraft sicher auf dem Dom 7 der Kappe 6.

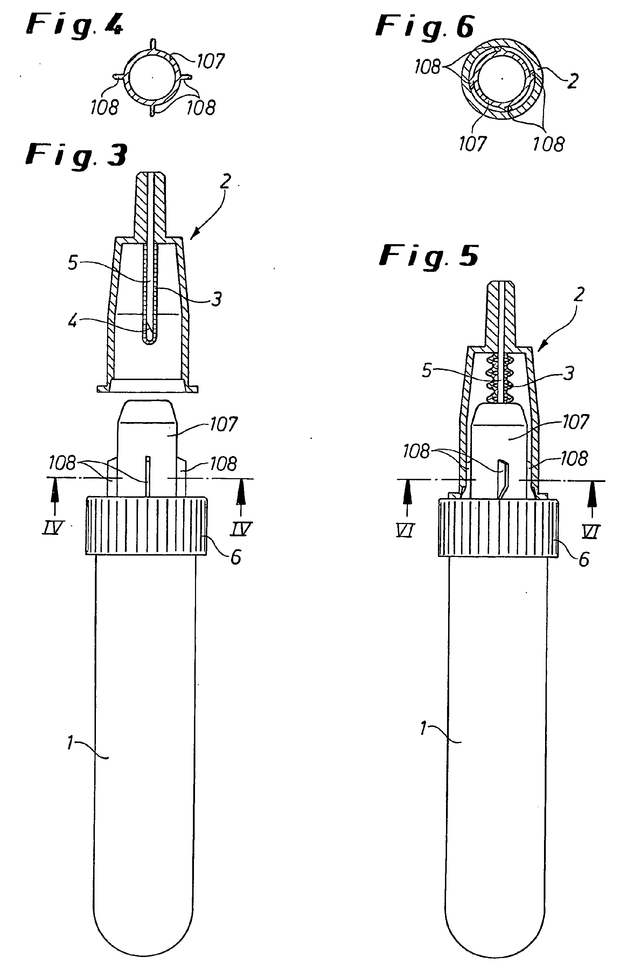

Diese Haltewirkung stellt sich auch dann ein, wenn gemäß der Ausführung nach den Figuren 3 bis 6 - mit der vorbeschriebenen Ausführung übereinstimmende Bauteile sind hier mit denselben Bezugsziffern versehen - der Dom 107 beim Aufstecken der verformungssteifen Führungshülse 2 seine ursprüngliche Kontur bzw. Form einhält und statt dessen die Haltemittel bzw. Längsrippen 108 so gestaltet sind, daß allein sie beim Aufstecken der Führungshülse 2 ausweichen, sich beispielsweise in Umfangsrichtung des Domes relativ zur Domwandung bzw. zum Innenmantel der Führungshülse 2 seitlich wegdrücken bzw. um ihre Längsachse 9 wegklappen, wie deutlich in den Figuren 5 und 6 gezeigt. Der Dom 107 ist hierbei wie die Führungshülse 2 verformungssteif, während die Haltemittel 108 elastisch nachgiebig sind.

Den Ausführungen nach den Figuren 1 und 2 sowie 3 bis 6 ist gemeinsam, daß bei stets gleichbleibendem Innendurchmesser der Führungshülse bei deren Aufstecken auf die Stopfenkappe aufgrund der Materialeigenschaften und maßlichen Abstimmung der im unbelasteten Zustand einschließlich der Haltemittel gegenüber dem Führungshülsen-Innendurchmesser ein Übermaß aufweisende Außendurchmesser des Doms sich verkleinert, indem entweder der Dom im Verbindungsbereich radial nach innen ausweicht oder die Haltemittel seitlich ausweichen. Die erforderliche Haltekraft läßt sich aber auch dann aufbringen, wenn der Innendurchmesser der verformungssteifen Führungshülse partiell kleiner als der Außendurchmesser des Doms ist, so daß dessen Wandung unverändert ebenfalls nach innen ausweicht, oder der Dom mit einer vorstehenden Kontur (z.B. bauchig oder tonnenartig) ausgebildet ist.

## Patentansprüche

1. Vorrichtung zur Aufnahme von Körperflüssigkeiten, mit einer Führungshülse (2) und einem Entnahmeröhrchen (1), das an seinem vorderen Ende mit einer Kappe (6) verschlossen ist, die mit einem zylindrischen Dom (7) mit einem durchstechbaren Stopfen für die aufsteckbare Führungshülse (2) versehen ist, die auf der dem Dom zugewandten Seite eine Kanüle (5) mit einem Ventilgummi (3) und auf der dem Dom abgewandten Seite ein Verbindungsstück oder den vorderen Teil einer Doppelkanüle aufweist,
**dadurch gekennzeichnet,**
**dass** der Dom (7) einen nach innen nachgiebigen Bereich aufweist und die Domaußenwandung in diesem Bereich und die Innenwand der verformungssteif ausgebildeten Führungshülse (2) in ihrem komplementären Bereich mit einer in Relation zum restlichen Außenbereich des Doms (7) und zum restlichen Innenbereich der Führungshülse (2) vorstehenden Kontur ausgebildet ist, die beim Aufstecken der verformungssteifen Führungshülse (2) die Wandung des Doms (7) radial nach innen verformt, wodurch in der zusammengefügten Gebrauchslage von Dom und Führungshülse eine kraftschlüssige Verbindung besteht, deren Haltekraft größer ist als die Rückstellkraft des Ventilgummis (3).

2. Vorrichtung zur Aufnahme von Körperflüssigkeiten, mit einer Führungshülse (2) und einem Entnahmeröhrchen (1), das an seinem vorderen Ende mit einer Kappe (6) verschlossen ist, die mit einem zylindrischen Dom (7) mit einem durchstechbaren Stopfen für die aufsteckbare Führungshülse (2) versehen ist, die auf der dem Dom zugewandten Seite eine Kanüle (5) mit einem Ventilgummi (3) und auf der dem Dom abgewandten Seite ein Verbindungsstück oder den vorderen Teil einer Doppelkanüle aufweist,
**dadurch gekennzeichnet,**
**dass** der Dom (7) einen nach innen nachgiebigen Bereich aufweist und die Innenwand der verformungssteif ausgebildeten Führungshülse (2) in ihrem zu dem nachgiebigem Teil des Doms (7) komplementären Bereich mit einer in Relation zum restlichen Innenbereich der Führungshülse (2) vorstehenden Kontur ausgebildet ist, die beim Aufstecken der verformungssteifen Führungshülse, (2) die Wandung des Doms (7) radial nach innen verformt, wodurch in der zugesammengefügten Gebrauchslage von Dom und Führungshülse eine kraftschlüssige Verbindung besteht, deren Haltekraft größer ist als die Rückstellkraft des Ventilgummis (3).

3. Vorrichtung zur Aufnahme von Körperflüssigkeiten, mit einer Führungshülse (2) und einem Entnahmeröhrchen (1), das an seinem vorderen Ende mit einer Kappe (6) verschlossen ist, die einen zylindrischen Dom (107) mit einem durchstechbaren Stopfen besitzt, wobei der Dom (107) an seinem Außenumfang mindestens ein radial abstehendes Haltemittel (108) für die auf den Dom aufsteckbare Führungshülse (2) trägt, die auf der dem Dom zugewandten Seite eine Kanüle (5) mit einem Ventilgummi (3) und auf der dem Dom abgewandten Seite ein Verbindungsstück oder den vorderen Teil einer Doppelkanüle aufweist,
**dadurch gekennzeichnet,**
**dass** der Dom (107) einschließlich mindestens einem Haltemittel (108) in einem Verbindungsbereich bezogen auf den Innendurchmesser der verformungesteifen Führungshülse (2) mit einem Übermaß ausgebildet ist und die Haltemittel (108) von einer solchen Beschaffenheit sind, dass sie beim Aufstecken der verformungssteifen Führungshülse (2) seitlich ausweichen und in der zusammengefügten Gebrauchslage von Führungshülse (2) und Dom (107) eine Haltekraft größer als die Rückstellkraft des Ventilgummis (3) aufbringen.

## Claims

1. A device for collecting body fluids, having a guidance sleeve (2) and a collection tube (1), the front end of which is sealed with a cap (6) that is furnished with a cylindrical spike (7) with a plug that may be pierced for the attachable guidance sleeve (2), which has a cannula (5) with a valve rubber (3) on the side facing the spike, and a connecting element or the front part of a double cannula on the side facing away from the spike,
**characterized in that**
the spike (7) has a zone that is flexible inwardly and the outer wall of the spike in this zone and the matching zone of the inner wall of the rigid guidance sleeve (2) is constructed with a contour that protrudes relative to the rest of the outer zone of the spiked 7) and the rest of the inner zone of the guidance sleeve (2), which deforms the wall of the spike (7) radially inwardly when the rigid guidance sleeve (2) is fitted, such that when the spike and the guidance sleeve are assembled and in the operating position a non-positive connection is created whose retaining force is greater than the restoring force of the valve rubber (3).

2. The device for collecting body fluids, having a guidance sleeve (2) and a collection tube (1), the front end of which is sealed with a cap (6) that is furnished with a cylindrical spike (7) with a plug that may be pierced for the attachable guidance sleeve (2), which has a cannula (5) with a valve rubber (3) on the side facing the spike, and a connecting element or the front part of a double cannula on the side facing away from the spike,
**characterized in that**
the spike(7) has a zone that is flexible inwardly and the inner wall of the rigidly constructed guidance sleeve (2) is constructed in the zone thereof that matches the yielding portion of the spike (7) with a protruding contour relative to the rest of the inner zone of the guidance sleeve (2), which deforms the wall of the spike (7) radially inwardly when the rigid guidance sleeve (2) is fitted, so that when the spike and the guidance sleeve are assembled and in the operating position a non-positive connection is created whose retaining force is greater than the restoring force of the valve rubber (3).

3. The device for collecting body fluids, having a guidance sleeve (2) and a collection tube (1), the front end of which is sealed with a cap (6) that is furnished with a cylindrical spike (107) with a plug that may be pierced, wherein the outer circumference of the spike (10&) is equipped with at least one radially projecting retaining means (108) for the guidance sleeve (102) that is attachable to the spike, which has a cannula (5) with a valve rubber (3) on the side facing the spike, and a connecting element or the front part of a double cannula on the side facing away from the spike,
**characterized in that**
the spike (107) including at least one retaining means (108) is oversized relative to the internal diameter of the rigid guidance sleeve (02) in a connecting zone, and the retaining means (108) are of such composition that they give way to the side when the rigid guidance sleeve (2) is attached, and apply a retaining force greater than the restoring force of the valve rubber (3) when the guidance sleeve (2) and the spike (3) are assembled and in the operating position.

## Revendications

1. Dispositif récepteur de liquides physiologiques, comportant un manchon de guidage (2) et une pipette de prélèvement (1) qui est fermée à son extrémité avant par un capuchon (6) qui est pourvu d'un dôme cylindrique (7) comprenant un bouchon traversable pour le manchon de guidage pouvant être fiché dessus (2) et qui présente sur sa face tournée vers le dôme une canule (5) à soupape de caoutchouc (3) et sur sa face détournée du dôme une pièce de raccordement ou la partie avant d'une double canule,
**caractérisé en ce que**
le dôme (7) présente une zone souple tournée vers l'intérieur et que la paroi extérieure du dôme dans cette zone et la paroi intérieure du manchon de guidage (2) réalisé de manière à être rigide à la déformation, dans sa zone complémentaire, est réalisée avec un contour proéminent par rapport au reste de la zone extérieure du dôme (7) et au reste de la zone intérieure du manchon de guidage (2), lequel contour, lorsqu'on fiche le manchon de guidage rigide à la déformation (2) déforme radialement vers l'intérieur la paroi du dôme (7), ce qui, en position assemblée d'utilisation du dôme et du manchon de guidage, établit un raccordement en correspondance mécanique dont la force de retenue est supérieure à la force de rappel de la soupape en caoutchouc (3).

2. Dispositif récepteur de liquides physiologiques, comportant un manchon de guidage (2) et une pipette de prélèvement (1) qui est fermée à son extrémité avant par un capuchon (6) qui est pourvu d'un dôme cylindrique (7) comprenant un bouchon traversable pour le manchon de guidage pouvant être fiché dessus (2) et qui présente sur sa face tournée vers le dôme une canule (5) à soupape de caoutchouc (3) et sur sa face détournée du dôme une pièce de raccordement ou la partie avant d'une double canule,
**caractérisé en ce que**
le dôme (7) présente une zone souple tournée vers l'intérieur et que la paroi intérieure du manchon de guidage (2) réalisé de manière à être rigide à la déformation est réalisée dans sa zone complémentaire à la partie souple du dôme (7) avec un contour proéminent par rapport au reste de la zone intérieure du manchon de guidage (2), lequel contour, lorsqu'on fiche le manchon de guidage rigide à la déformation (2) déforme radialement vers l'intérieur la paroi du dôme (7), ce qui, en position assemblée d'utilisation du dôme et du manchon de guidage, établit un raccordement en correspondance mécanique dont la force de retenue est supérieure à la force de rappel de la soupape en caoutchouc (3).

3. Dispositif récepteur de liquides physiologiques, comportant un manchon de guidage (2) et une pipette de prélèvement (1) qui est fermée à son extrémité avant par un capuchon (6) qui est pourvu d'un dôme cylindrique (107) comprenant un bouchon traversable, le dôme (107) supportant sur sa périphérie extérieure au moins un moyen de retenue proéminent radialement (108) pour le manchon de guidage (2) pouvant être fiché sur le dôme, lequel manchon de guidage comporte, sur sa face tournée vers le dôme une canule (5) à soupape de caoutchouc (3) et sur sa face détournée du dôme une pièce de raccordement ou la partie avant d'une double canule,
**caractérisé en ce que**
le dôme (107), y compris au moins un moyen de retenue (108), est réalisé dans une zone de raccordement avec une surcote par rapport au diamètre intérieur du manchon de guidage rigide à la déformation (2) et que les moyens de retenue (108) sont réalisés de manière à ce que, lorsqu'on fiche le manchon de guidage rigide à la déformation (2), ils s'écartent latéralement et, en position d'utilisation assemblée du manchon de guidage (2) et du dôme (107), ils appliquent une force de retenue supérieure à la force de rappel de la soupape en caoutchouc (3).
